# EUROPEAN PATENT APPLICATION

(11) **EP 4 470 542 A1**
(43) Date of publication of application: **04.12.2024**
(21) Application number: 23382523.1
(22) Date of filing: 31.05.2023
(51) Int. Cl.: A61K 31/7048, A61K 39/00, A61K 45/06, A61P 35/00

(54) **OLEUROPEIN IN THE MANAGEMENT OF CANCER**

(71) Applicant: Fundación Miguel Servet, 31008 Pamplona (ES); Fundación Instituto de Investigación Sanitaria de Navarra, 31008 Pamplona (ES); Universidad Pública De Navarra, 31006 Pamplona (ES)
(72) Inventor: ESCORS MURUGARREN, David, 31008 Pamplona (ES); BLANCO PALMEIRO, Ester, 31008 Pamplona (ES); CHOCARRO DE ERAUSO, Luisa, 31008 Pamplona (ES); KOCHAN, Grazyna, 31008 Pamplona (ES)
(74) Representative: Hoffmann Eitle

(57) **Abstract**

The present invention provides the use of oleuropein in immunotherapy, particularly as adjuvant. Particularly, the invention provides the use of oleuropein in combination with a checkpoint inhibitor in the treatment of cancer.

The invention also provides combinations comprising the oleuropein and the checkpoint inhibitor, as well as pharmaceutical compositions and kits of parts including the combination.

Advantageously, the oleuropein reprograms TAMCs, increasing the sensitivity towards anticancer therapies based on checkpoint inhibitors.

## Description

### FIELD OF THE INVENTION

The present invention is related to the field of medicine, in particular to the treatment of cancer. Concretely, the present invention provides the use of oleuropein, alone or in combination with one or more anti-cancer drug(s) in the treatment of cancer.

### BACKGROUND OF THE INVENTION

Tumor associated myeloid cells (TAMCs) are considered as major promoters of tumor progression and metastasis. Among these cell populations, myeloid-derived suppressor cells (MDSCs) together with tumor-infiltrating macrophages (TAMs) are the main contributors to tumor-induced immunosuppression. In addition, myeloid cells are the most abundant population in the tumor microenvironment (TME).

MDSCs are cells having immunosuppressive action among myeloid cells and as a cell population including very widely undifferentiated myeloid cells, are increased in a generation state of tumors or inflammation. It is known that these MDSCs have the immunosuppressive action through a direct contact between most of cells and it is understood that the immunosuppressive function is performed by secreting materials such as cytokines. But also an indirect contact, as the cytokine secretation. The immunosuppressive role of these cells induces tumor progression.

Systemic elevation of MDSC numbers in many cancer types, including non-small cell lung cancer (NSCLC), has been associated with resistance to chemotherapy, targeted therapy, and immunotherapy. Overall, this increase of immunosuppressive myeloid cells is considered as a poor prognostic factor. In fact, the activation status of these myeloid subsets could be used as predictor for clinical responses in patients treated with immune checkpoint blockers, as monoclonal antibodies (mAbs) against programmed death 1 (PD-1) or its ligand (PD-L1).In addition, increasing experimental evidence indicates that MDSC reprogramming in colon cancer takes place in responder patients after PD-1 blockade. All these studies suggest that reprogramming MDSCs before or during anti-PD-L1/PD-1 treatment may overcome resistance to this type of immunotherapy. Recent evidences indicate that metabolism is an important regulator of immune cell phenotype and function.

However, the particular molecular mechanisms regulating their pro- or antitumor properties are still under investigation because isolation of specific myeloid cell subsets from the tumor microenvironment presents many technical challenges.

On the other hand, the methods of suppressing MDSCs indicate a possibility that the anticancer immunotherapy achieves a clinical effect, but do not yet achieve the treatment results to reduce a significant change in survival rate of the patient. Particularly, a drug such as the gemcitabine in the related art simultaneously reduces the MDSC and the CD3 T cells and thus immunocompetence of cancer patients is seriously deteriorated to have a negative effect on the prognosis of the cancer patients. Further, in the cancer patients, at the time of primarily removing the MDSC and additionally treating secondary immunity, when the number of T lymphocytes in the body of the patient is decreased, the effect of the immunotherapy is very low and thus the retention of the T lymphocytes is absolutely required when removing the MDSC by the drug.

In spite of the efforts made, therefore, there is still the need of further therapies that allow the efficient treatment of cancer.

### SUMMARY OF THE INVENTION

A major goal in oncoimmunology is the reprogramming of immunosuppressive myeloid cells towards immunogenic subsets that can activate anti-tumour responses.

In this context, the inventors have found that oleuropein causes a major reprogramming of intracellular pathways in cancer-associated immunosuppressive myeloid cells towards stimulatory myeloid antigen presenting cells. This is indicative of the immunostimulatory effect that oleuropein provides in cancer subjects.

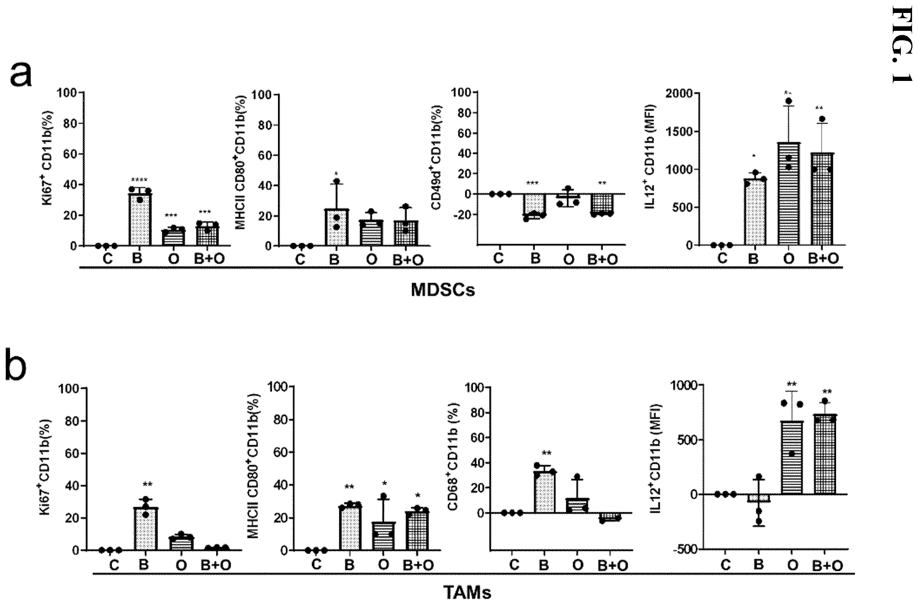

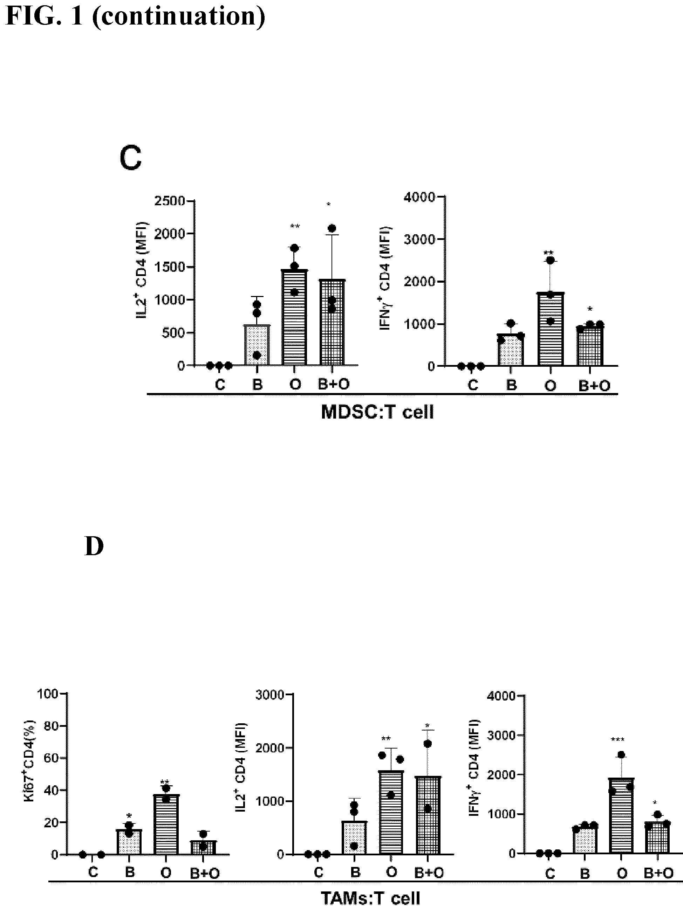

As it is shown below, the reprogramming effect resulted in enhanced T cell activation capacities, exemplified by up-regulation of antigen presentation markers (MHC-II and CD80) and induction of IL-12 (Fig. 1 A, B, C, D). The reprogramming was demonstrated in the three main immunosuppressive myeloid populations at high purities: tumor-associated macrophages, granulocytic MDSCs and monocytic MDSCs.

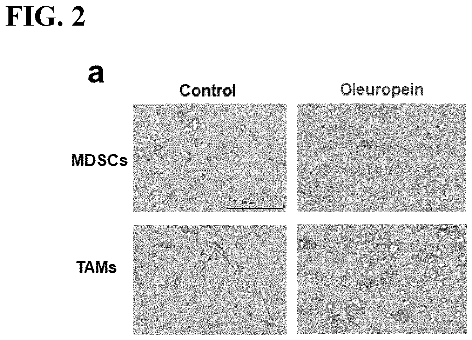

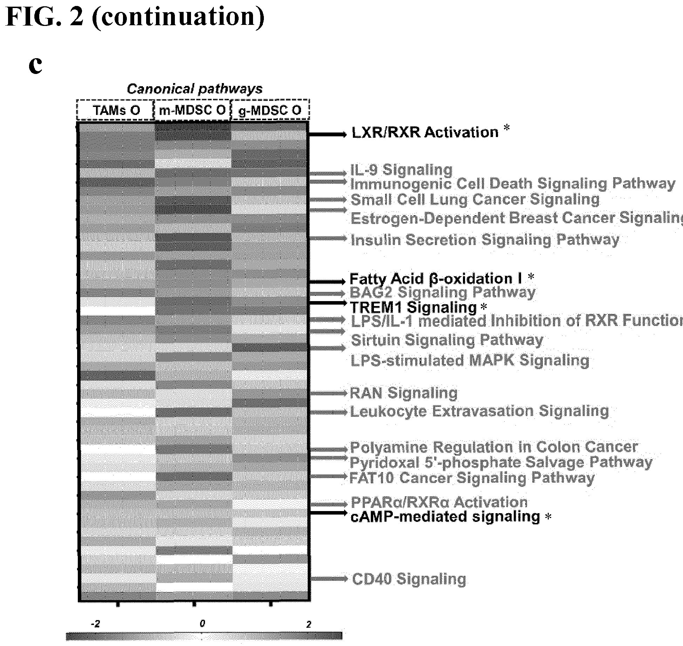

One of the activated pathways was LXR/RXR signalling (Fig. 2c), for which there was no previous evidence on its regulation by oleuropein or any other polyphenol. LXR/RXR is known as being involved in lipid and glucose metabolism, as well as in inflammatory responses. The inventors confirmed that this LXR agonism reduced the abundance of MDSC subsets *in vivo* as well as of immunosuppressive TME.

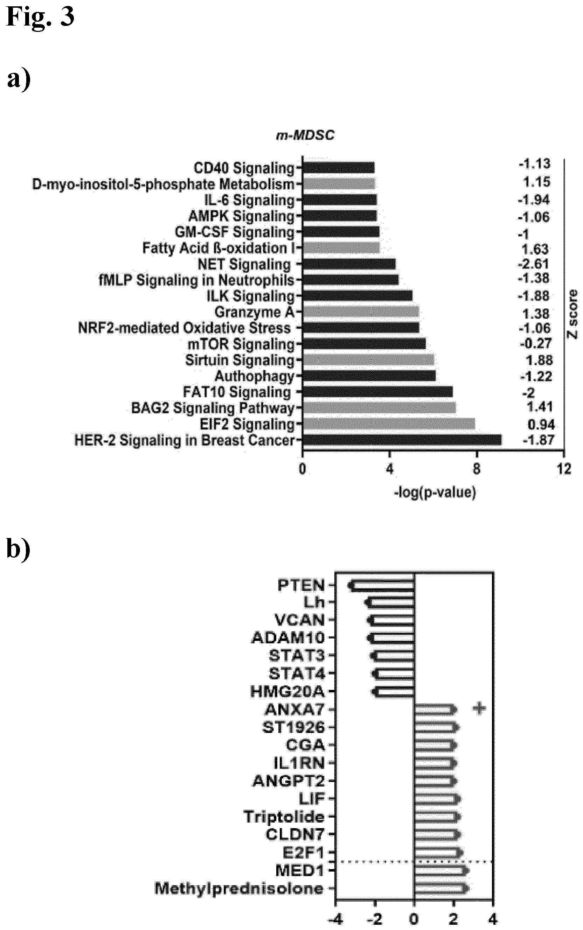

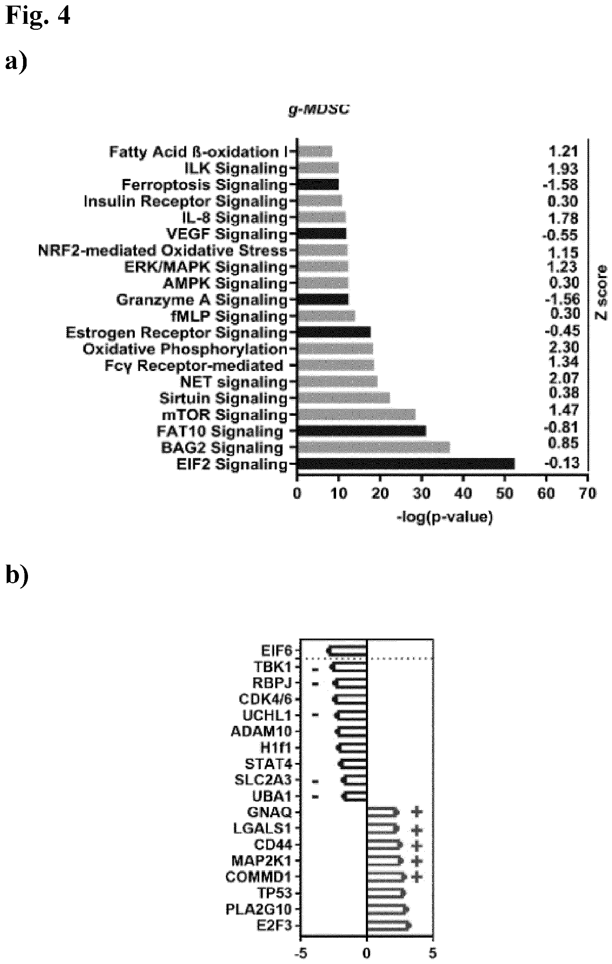

Moreover, the administration of oleuropein also provided unexpected properties on MDSCs differentiation. Oleuropein demonstrated a potent effect over m-MDSC intracellular regulatory pathways by modulating transcription factors involved in MDSCs differentiation. In the case of granulocytic-MDSC treated with oleuropein, these tumor-associated immunosuppressive myeloid cells drastically changed their cellular programming, acquiring an entirely different proteome following oleuropein treatment, highlighting immunosuppressive markers and transcription factors (Fig. 3b,4b).

Likewise, TREM-1 and cAMP inhibition were also discovered in myeloid cells treated with oleuropein (Fig. 2c).

TREM-1 and cAMP are being involved in myeloid cell regulation. In the particular case of TREM-1, the prior art has already related the TREM-1 expression in TAM-associated with HCC resistance to anti-PD-L1 therapy. Interestingly, oleuropein inhibited TREM1 and cAMP in all TAMCs, which is indicative of the potential value of oleuropein in reverting cancer resistance to anti-PD-1 and anti-PD-L1 therapies, thus improving the survival and quality of life of cancer patients; but also of in counteracting the inflammatory adverse events frequently associated to PD-L1/PD-1 blockade.

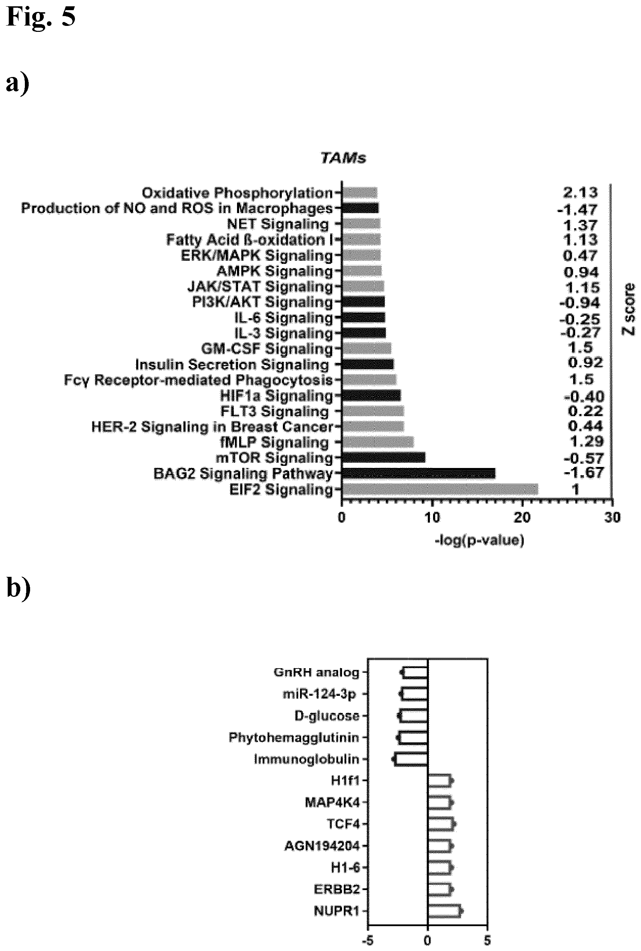

It was also found that oleuropein polarized TAMs to activated macrophages, underlining the upregulation of Fcr-mediated phagocytosis signalling and the downregulation of immunosuppressive markers (Fig. 5a). The systemic elimination of immunosuppressive myeloid cells will have an overall positive impact by counteracting cachexia.

Finally, it was also confirmed that oleuropein induced the activation of myeloid cells of subjects suffering lung cancer (including those suffering from anti-PD-1-based therapy resistance).

Altogether, the present invention means a great advance in the management of cancer.

Thus, in a first aspect the present invention provides the oleuropein for use in immunotherapy, particularly as immunotherapy adjuvant.

In a second aspect the present invention provides the oleuropein for use as immunogen in a patient suffering from cancer.

In a third aspect the present invention provides the oleuropein for use in inducing immunogenicity to immunosuppressive cells, particularly to immunosuppressive tumor associated myeloid cells (TAMCs) population, particularly to myeloid-derived suppressor cell (MDSCs) and/or tumor-associated macrophage (TAMs) population.

As it is shown below, oleuropein exhibited potent antitumor capacities and most importantly, enhanced anti-PD-1 therapies in models of lung and colon cancer. Importantly, oleuropein improved αPD-1 therapies in a tumor model intrinsically resistant to PD-1 blockade highlights the potentiality of this therapy for cancer treatment. Moreover, these pre-clinical results lead us to put forward oleuropein as a potential adjuvant for clinical trials.

In a fourth aspect the present invention provides the oleuropein for use in a method for the prevention or treatment of cancer in combination with one or more anti-cancer drugs, wherein at least one of the anti-cancer drugs is a checkpoint inhibitor. This aspect can alternatively be formulated as the use of oleuropein for the manufacture of a medicament for the prevention or treatment of cancer in combination with one or more anti-cancer drugs, wherein at least one of the anti-cancer drugs is a checkpoint inhibitor. This aspect can also be formulated as a method for the prevention or treatment of cancer, the method comprising the step of administering a therapeutically effective amount of oleuropein in combination with one or more anti-cancer drugs, wherein at least one of the anti-cancer drugs is a checkpoint inhibitor, to a subject in need thereof.

In a fifth aspect the present invention provides a combination comprising oleuropein and a checkpoint inhibitor anti-cancer drug.

In a sixth aspect the present invention provides a pharmaceutical composition comprising the combination according to the fifth aspect of the invention, and one or more pharmaceutically acceptable excipients and/or carriers.

In a seventh aspect the present invention provides a kit of parts comprising:
- Oleuropein,
- A checkpoint inhibitor anti-cancer drug, and
- Optionally carriers or excipients to prepare separate pharmaceutical composition(s).

In final aspects the present invention also provides the combination, pharmaceutical composition and kit of parts as defined in the fifth, sixth, and seventh aspects of the invention, for use in any of the aspects first to third defined above.

### BRIEF DESCRIPTION OF THE DRAWINGS

Fig. 1. Immunomodulatory properties of BML-275 and Oleuropein in MDSCs and TAMs. (a)Percentage of MDSCs differentiated ex vivo that express Ki67 CD11b, CD11b MHCII CD80, CD11b CD49d and CD11b IL-12. (b) Percentage of TAMs differentiated ex vivo that express Ki67 CD11b, CD11b MHCII CD80, CD11b CD68 and CD11b IL-12. (c,d) Bar graphs represent Ki-67, IL-2 and IFN-γ expression within CD4 cells in MLR using either MDSC as stimulator cells or TAMs, treated or untreated with BML-275, Oleuropein as indicated. Control (C)BML-275 (B) (500nM), and Oleuropein (O) (50uM). Means and SD are presented in the bar graphs. Data in (a-d) are representative of at least two independent experiments in triplicate wells (mean ± SD) and analyzed by a two-tailed Student's t test.

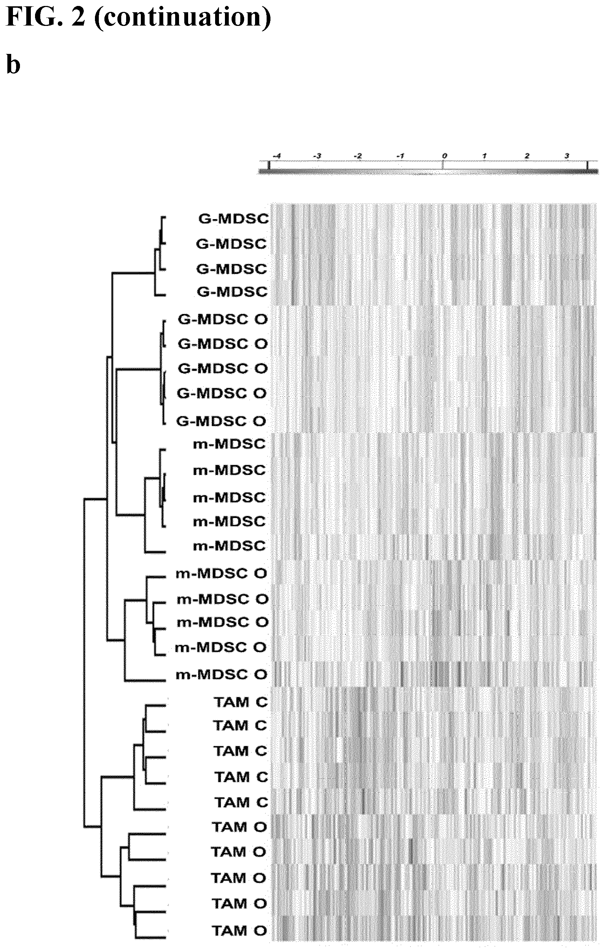

Fig. 2. Differential pathways modulated in TAMCs by oleuropein (a) Phase contrast micrographs of ex vivo cultures of MDSCs and TAMs treated with oleuropein. (b) Hierarchical unbiased clustering representing the differentially expressed proteins between m-MDSC, g-MDSC, and TAM treated or nor with oleuropein (O) using 4-5 cell culture replicates (two sample test, P<0.01). (c) Canonical pathways enriched in m-MDSC, g-MDSCs, and TAMs treated with oleuropein compared with untreated cells. Data represented in heatmaps according to Z-score of expressed genes predicted to be involved in canonical pathways. Z-score: red and blue coloured indicate predicted activation or inhibition, respectively.
Fig. 3. Differential pathways modulated in m-MDSC by oleuropein. (a) Canonical pathways enriched in m-MDSC treated with oleuropein compared with untreated cells. Data represented in bar chart according to -log(p-value): probability of association of molecules (P<0.05) and Z-score: grey and black-colored bars indicate predicted pathway activation or predicted inhibition, respectively. Data was analyzed by IPA tool. (b) Main upstream regulators in m-MDSC treated with oleuropein. The graph represents the Z-score: probability of each indicated transcription factor to be either activated (right graph) or inhibited (left graph) in m-MDSCs treated with oleuropein using the IPA tool.
Fig. 4. Differential pathways modulated in g-MDSC by oleuropein. a) Canonical pathways enriched in g-MDSCs treated with oleuropein compared with untreated cells. Data represented in bar chart according to -log(p-value): probability of association of molecules (P<0.05) and Z-score: grey and blackcolored bars indicate predicted pathway activation or predicted inhibition, respectively. Data was analyzed by IPA tool. b) Main upstream regulators in g-MDSC treated with oleuropein. The graph represents the Z-score: probability of each indicated transcription factor to be either activated (right graph) or inhibited (left graph) in g-MDSCs treated with oleuropein using the IPA tool.
Fig. 5. Differential pathways modulated in TAM by oleuropein. a) Canonical pathways enriched in TAM treated with oleuropein compared with untreated cells. Data represented in bar chart according to -log(p-value): probability of association of molecules (P<0.05) and Z-score: orange and blue-colored bars indicate predicted pathway activation or predicted inhibition, respectively. Data was analyzed by IPA tool. b) Main upstream regulators in TAM treated with oleuropein. The graph represents the Z-score: probability of each indicated transcription factor to be either activated (right graph) or inhibited (left graph) in TAM treated with oleuropein using the IPA tool.

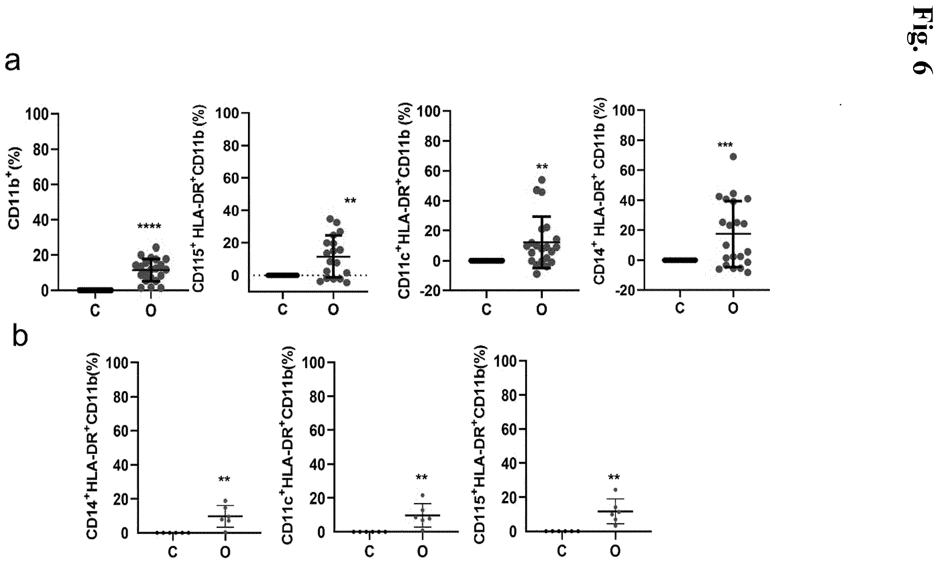

Fig. 6 (a) Myeloid population profile of NSCLC patients changed by oleuropein (50uM) compared to control non-treated cells (black). Graphs show the percentage of myeloid cells from NSCLC patients that express the indicated markers normalized to the control, N=21).

The Shapiro wilk test was used for normality followed by Student's paired-tailed t-test *, p<0.05; **, p<0.01; ***, p<0.001; ****, p<0.0001. (b) Myeloid population profile of healthy donors changed by oleuropein (50µM). Percentage of myeloid cells from healthy donors that express the indicated markers normalized to the control (n=6), oleuropein N=6). The one sample t test and Wilcoxon test was used and mean ± SD is represented *, p<0.05; **, p<0.01; ***, p<0.001; ****, p<0.0001. Control, black and oleuropein, red.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As mentioned above, the invention provides, in a first aspect, the use of oleuropein in immunotherapy.

In the context of the invention, "immunotherapy" means treating or preventing a disease or condition by methods that involve inducing, enhancing, suppressing, or otherwise modulating an immune response.

Oleuropein is an ester of 2'-(3',4'-dihydroxyphenyl)ethanol (hydroxytyrosol) and the oleosidic skeleton common to the secoiridoid glucosides, that has been identified in the leaves of Olea europaea, as well as in the bark and roots of the tree. It is a bitter glycoside that decomposes easily, by acid or enzymic hydrolysis, to elenolic acid and glucose. Oleuropein is present in high amounts (60-90 mg/g dry weight) in the leaves of the olive tree, but it has been found throughout the tree, including all constituent parts of the fruit (peel, pulp and seed). Methods of preparing extracts from olive leaves or olive fruits as a source for oleuropein are well-known to those skilled in the art. An illustrative example, based on microwave radiation, is provided in detail below.

Alternatively, the oleuropein is also commercially available, and can be purchased e.g. from Extrasynthese (France), in the form of its glucoside.

The term "oleuropein" encompasses any olive leaf extract and chemical derivative thereof provided that it retains the beneficial effects reported in the present invention (cf. Nardi M et al., "Synthesis and antioxidant evaluation of lipophilic oleuropein aglycone derivatives", Food Funct., 2017;8(12):4684-4692).

In one embodiment, the oleuropein is obtained by a process comprising placing olive leaves in water under microwave radiation. In one embodiment of this process, the olive leaves have been previously dried. The skilled person in the art will be able to adjust the potency of the microwave and time in view of the amount of dried olive leaves (cf., M. Oliverio et al., "Semi-synthesis as a tool for broadening the health applications of bioactive olive secoiridoids: a critical review", Natural Product Reports, 2021, 38, 444-469). In one embodiment, the potency of the microwave radiation is from 500 to 1000 W, and the time of exposure from 5 to 20 min.

The amount of oleuropein to be administered to the subject is within the range from 10-90 mg, particularly from 50-80 mg/day.

In a second aspect the invention provides the use of oleuropein as immunogen in a cancer patient.

In the context of the invention, the term "immunogen" means a substance that induces a specific immune response in a host animal.

In a further aspect the invention provides the combined use of oleuropein and at least one checkpoint inhibitor.

Inhibitory checkpoint pathways involve signalling through the cytotoxic T-lymphocyte antigen-4 (CTLA-4) and programmed-death 1 (PD-1) receptors. These proteins are members of the CD28-B7 family of co-signalling molecules that play important roles throughout all stages of T cell function. The PD-1 receptor (also known as CD279) is expressed on the surface of activated T cells. Its ligands, PD-L1 (B7-H1; CD274) and PD-L2 (B7-DC; CD273), are expressed on the surface of APCs such as dendritic cells or macrophages. PD-L1 is the predominant ligand, while PD-L2 has a much more restricted expression pattern. When the ligands bind to PD-1, an inhibitory signal is transmitted into the T cell, which reduces cytokine production and suppresses T-cell proliferation. A list of checkpoint targeting antibodies suitable in the context of the invention are provided in Table1.

| Table 1. Clinically evaluated immune-checkpoint blocking antibodies | |
|---|---|
| Target | Antibody |
| CTLA-4 | Ipilimumab |
| | (MDX-010) |
| | Tremelimumab |
| | (CP-675,206) |
| PD1 | Nivolumab (BMS-936558 or MDX1106) |
| | CT-01 1 |
| | MK-3475 |
| PDL1 | MDX-1105 (BMS-936559) |
| | MPDL3280A |
| | MSB0010718C |
| PDL2 | rHIgM12B7 |
| B7-H3 | MGA271 |
| LAG3 | BMS-986016 |

Human monoclonal antibodies to programmed death 1 (PD-1) and methods for treating cancer using anti-PD-1 antibodies alone or in combination with other immunotherapeutics are described in US8008449. Anti-PD-L1 antibodies and uses therefor are described in US8552154. Anticancer agent comprising anti-PD-1 antibody or anti-PD-L1 antibody are described in US8617546.

The term "antibody" refers to natural or synthetic antibodies that selectively bind a target antigen. The term includes polyclonal and monoclonal antibodies. In addition to intact immunoglobulin molecules, also included in the term "antibodies" are fragments or polymers of those immunoglobulin molecules, and human or humanized versions of immunoglobulin molecules that selectively bind the target antigen.

"Antibody fragments" refers to a portion of an immunoglobulin molecule that retains the heavy chain and/or the light chain antigen binding site, such as heavy chain complementarity determining regions (HCDR) 1, 2 and 3, light chain complementarity determining regions (LCDR) 1, 2 and 3, a heavy chain variable region (VH), or a light chain variable region (VL). Antibody fragments include a Fab fragment, a monovalent fragment consisting of the VL, VH, CL and CHI domains; a F(ab)₂ fragment, a bivalent fragment comprising two Fab fragments linked by a disulfide bridge at the hinge region; a Fd fragment consisting of the VH and CHI domains; a Fv fragment consisting of the VL and VH domains of a single ann of an antibody; a domain antibody (dAb) fragment (Ward et a!., Nature 341:544- 6, 1989), which consists of a VH domain. VH and VL domains may be engineered and linked together via a synthetic linker to form various types of single chain antibody designs in which the VH/VL domains pair intramolecularly, or intennolecularly in those cases when the VH and VL domains are expressed by separate single chain antibody constructs, to form a monovalent antigen binding site, such as single chain Fv (scFv) or diabody. These antibody fragments are obtained using well known techniques known to those of skill in the art, and the fragments are screened for utility in the same manner as are full length antibodies.

The combination can also include one or more further anti-cancer agents, selected from chemotherapeutic agents, targeted therapeutic agents, antibody therapeutic agents, further immunotherapeutic agents and hormone therapeutic agents.

The immunotherapeutic agent includes an agent designed to induce a self-immune system of a subject in order to attack the tumor, for example, ipilimumab, avelumab, nivolumab, and pembrolizumab, but is not limited thereto.

The hormone therapeutic agent includes an agent of suppressing a growth of the cancer by providing or interrupting a hormone in a specific cancer, for example, tamoxifen and diethylstilbestrol, but is not limited thereto.

The appropriate dose of the anti-cancer agents is widely known in the art and thus may be administrated by a known reference in the art according to a state of each patient. A particular dose is determined within a level of those skilled in the art, and a daily dose thereof is particularly 1 mg/kg/day to 10 mg/kg/day and more particularly 10 mg/kg/day to 100 mg/kg/day, but is not limited thereto and may vary according to various factors including age, health status, complications, and the like of a subject to be administrated.

Numerous anti-cancer drugs are available and suitable in the context of the invention. The following is a non-exhaustive lists of anti-cancer (anti-neoplastic) drugs that can be used in conjunction with irradiation: Acivicin; Aclarubicin; Acodazole Hydrochloride; AcrQnine; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflomithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Ethiodized Oil I 131; Etoposide; Etoposide Phosphate; Etoprine; Fadrozole Hydrochloride; Fazarabine; Fenretinide; Floxuridine; Fludarabine Phosphate; Fluorouracil; Flurocitabine; Fosquidone; Fostriecin Sodium; Gemcitabine; Gemcitabine Hydrochloride; Gold Au 198; Hydroxyurea; Idarubicin Hydrochloride; Ifosfamide; Ilmofosine; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safmgol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Strontium Chloride Sr 89; Sulofenur; Talisomycin; Taxane; Taxoid; Tecogalan Sodium; Tegafur; Teloxantrone Hydrochloride; Temoporfin; Teniposide; Teroxirone; Testolactone; Thiamiprine; Thioguanine; Thiotepa; Tiazofurin; Tirapazamine; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride.

The oleuropein as well as the anti-cancer drug(s) can be administered in the form of separate pharmaceutical compositions or, alternatively, in a single pharmaceutical composition.

The present invention further provides a pharmaceutical composition comprising the combination oleuropein and at least one checkpoint inhibitor, together with any of the embodiments provided above (regarding the oleuropein and anti-cancer drug(s)).

The expression "pharmaceutical composition" encompasses both compositions intended for human as well as for non-human animals (i.e. veterinarian compositions).

The term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion).

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Examples of suitable aqueous and nonaqueous carriers that may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminium monostearate and gelatine.

The term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio.

Pharmaceutically acceptable carriers also include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Pharmaceutical compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of oleuropein or anti-cancer agent to be combined with a carrier material to produce a single dosage form will vary depending upon the subject being treated, and the particular mode of administration. The amount of ingredient which can be combined with a carrier material to produce a single dosage form will generally be that amount of the composition which produces a therapeutic effect. Generally, out of one hundred percent, this amount will range from about 0.01 percent to about ninety-nine percent of active ingredient, preferably from about 0.1 percent to about 70 percent, most preferably from about 1 percent to about 30 percent of active ingredient in combination with a pharmaceutically acceptable carrier.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

Actual dosage levels of the active ingredients in the pharmaceutical compositions may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The compositions suitable in the context of the invention can be administered via one or more routes of administration using one or more of a variety of methods known in the art. As will be appreciated by the skilled artisan, the route and/or mode of administration will vary depending upon the desired results. Preferred routes of administration for antibodies of the invention include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, the composition can be administered via a non-parenteral route, such as a topical, epidermal or mucosal route of administration, for example, intranasally, orally, vaginally, rectally, sublingually or topically.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art.

In a further aspect the present invention provides a kit of parts including
- Oleuropein,
- One or more anti-cancer drugs, particularly at least one checkpoint inhibitor,
- Optionally carriers or excipients to prepare separate pharmaceutical composition(s).

All the embodiments provided above, regarding the oleuropein, anti-cancer drugs, checkpoint inhibitors, excipients and carriers are also embodiments of the kit of parts of the invention.

The term "cancer" or "malignant neoplasm" refers to a cell that displays uncontrolled growth, invasion upon adjacent tissues, and often metastasis to other locations of the body.

The term "metastasis" refers to the spread of malignant tumor cells from one organ or part to another non-adjacent organ or part. Cancer cells can "break away," "leak," or "spill" from a primary tumor, enter lymphatic and blood vessels, circulate through the bloodstream, and settle down to grow within normal tissues elsewhere in the body. When tumor cells metastasize, the new tumor is called a secondary or metastatic cancer or tumor.

The cancer of the disclosed methods can be any cell in a subject undergoing unregulated growth, invasion, or metastasis. Thus, the cancer can be a sarcoma, lymphoma, leukemia, carcinoma, blastoma, or germ cell tumor. A representative but non-limiting list of cancers that the disclosed compositions can be used to treat include lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, kidney cancer, lung cancers such as small cell lung cancer and non-small cell lung cancer, neuroblastoma/glioblastoma, ovarian cancer, pancreatic cancer, prostate cancer, skin cancer, liver cancer, melanoma, squamous cell carcinomas of the mouth, throat, larynx, and lung, colon cancer, cervical cancer, cervical carcinoma, breast cancer, epithelial cancer, renal cancer, genitourinary cancer, pulmonary cancer, esophageal carcinoma, head and neck carcinoma, large bowel cancer, hematopoietic cancers; testicular cancer; colon and rectal cancers, prostatic cancer, and pancreatic cancer. In some embodiments, the cancer is small cell lung cancer (SCLC).

The term "subject" refers to any individual who is the target of administration or treatment. The subject can be a vertebrate, for example, a mammal. Thus, the subject can be a human or veterinary patient. The term "patient" refers to a subject under the treatment of a clinician, e.g., physician.

The term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

A "therapeutically effective of oleuropein" preferably results in one or more of the following effects: reduction in the tumor associated myeloid cells (TAMCs) population, particularly myeloid-derived suppressor cell (MDSCs) and/or tumor-associated macrophage (TAMs) population; induction of immune cell activation; and increase in the sensitivity for the one or more anti-cancer drug(s).

A "therapeutically effective amount of the one or more anti-cancer agents" preferably results in a decrease in severity of disease symptoms, an increase in frequency and duration of disease symptom-free periods, or a prevention of impairment or disability due to the disease affliction. For example, for the treatment of tumors, a "therapeutically effective dosage" preferably inhibits cell growth or tumor growth by at least about 20%, more preferably by at least about 40%, even more preferably by at least about 60%, and still more preferably by at least about 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition in vitro by assays known to the skilled practitioner. A therapeutically effective amount of a therapeutic compound can decrease tumor size, or otherwise ameliorate symptoms in a subject. One of ordinary skill in the art would be able to determine such amounts based on such factors as the subject's size, the severity of the subject's symptoms, and the particular composition or route of administration selected.

In one embodiment of the fourth aspect of the invention, the oleuropein reduces the tumor associated myeloid cells (TAMCs) population, particularly myeloid-derived suppressor cell (MDSCs) and/or tumor-associated macrophage (TAMs) population.

In the present invention, the "myeloid-derived suppressor cells" function to suppress immunity by inhibiting activity of cytotoxic T lymphocyte. There is a positive function of suppressing unnecessarily excessive immune response such as autoimmune, but there is a negative function of generating or deteriorating diseases or interrupting proper treatment by suppressing immunity in a situation where immune response is required. For example, the myeloid-derived suppressor cells are largely increased in tumor or cancer patients, and this significantly reduces effects of administrating cancer therapy. In such a situation, when the number of myeloid-derived suppressor cells is effectively reduced, the cancer treatment may be smoothly and effectively performed.

In the present invention, the "reduction in the myeloid-derived suppressor cells" includes up to suppressing activity of the myeloid-derived suppressor cells as well as reducing the number of myeloid-derived suppressor cells. The reducing of the number includes killing pre-generated cells or differentiating the cells to other cells as well as suppressing generation of the cells. In addition, all mechanisms referred to as "inhibition" from the biological point of view are included.

The myeloid-derived suppressor cells of the subject having the tumor may be at least one selected from the group consisting of phenotypes of CD11b⁺Gr1^{hi}, CD11b⁺F480⁻Gr1⁺, CD11b⁺Ly6c^{hi}, CD11b⁺Ly6c⁺Ly6g⁺, CD11b⁺Ly6c⁺⁺Ly6g⁻, CD11b⁺Ly6c⁺⁺Ly6g⁻, CD11b⁺Lineage⁻(CD3, CD14, CD19 and CD56)HLA-DR⁻CD33⁺ and CD11b⁺CD14⁺HLA-DR⁻CD15⁻, more preferably phenotypes of CD11b⁺Gr1^{hi}, CD11b⁺F480⁻Gr1⁺, CD11b⁺Ly6c^{hi}, CD11b⁺Ly6c⁺Ly6g⁺ and CD11b⁺Ly6c⁺⁺Ly6g⁻ in mice, and CD11b⁺Ly6c⁺⁺Ly6g⁻, CD11b⁺Lineage⁻(CD3, CD14, CD19 and CD56)HLA-DR⁻CD33⁺ and CD11b+CD14⁺HLA-DR⁻CD15⁻ in human but are not limited thereto.

The function of TAMs can depend on their accumulation and activation in tumor tissues, therefore, TAM-targeted anti-tumor approaches can based on the following four strategies: 1) inhibiting macrophage recruitment; 2) suppressing TAM survival; 3) enhancing M1 tumoricidal activity of TAMs; and 4) blocking M2 tumor-promoting activity of TAMs.

Chemoattractants released by tumor cells can facilitate the recruitment of macrophages into tumor tissues. To suppress TAM survival, two approaches can be used. One can be to directly induce macrophage apoptosis using chemical reagents, immunotoxin-conjugated monoclonal antibodies or attenuated bacteria; the other can be to trigger the immune cells, for example T lymphocytes, to recognize and abrogate TAMs.

In the context of the invention oleuropein does not provide any apoptotic effect, as reported in detail below, but that reprograms these cell populations to act protecting, instead of attacking, to the organism. And this is due to the fact that both, oleuropein induces an immune cell activation, particularly T cell activation, more particularly CD4 T cell activation, as reported below.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

### MATERIALS AND METHODS

### Animal studies

Four-week-old female C57BL/6 mice were purchased from Envigo (Barcelona, Spain). Animal studies were approved by the University of Navarra ethics committee (E20-22(077-19).

### Cells

LLC cells were grown in DMEM (GIBCO BRL, UK) supplemented with 10% FBS, 2 mM glutamine, and antibiotics. LLC cells constitutively expressing mouse GM-CSF (LLC-GMCSF) were generated by lentivector transduction following the published methodologies [4].

MC38 were cultured in RPMI-1640 medium (Lonza, Switzerland) supplemented with 10% FBS, 2 mM glutamine, 20 mM HEPES, antibiotics and 50 µM 2-mercaptoethanol.

### Ex-vivo differentiation and purification of murine MDSCs and TAMs

Immunosuppressive myeloid cell subsets resembling tumor-infiltrating subsets (g-MDSCs, m-MDSCs and TAMs) were differentiated from mouse bone marrow following published procedures using conditioning medium produced by LLC tumor cells stably expressing GMCSF and MCSF cells [1]. g- and m-MDSCs were purified using the myeloid-derived suppressor cell isolation kit (Miltenyi Biotec, Bergisch,Germany) according to manufacturer's recommendations. MDSCs and TAMs were seeded with 500nM BML-275 or 50 µM oleuropein and incubated for seven days.

### Oleuropein Microwave-Assisted Extraction Procedure

Oleuropein was obtained using a novel strategy, as described by Procopio et al [4]. Briefly, 100 g of olive leaves of Olea europaea from Coratina cultivar, dried for 48 h at 50 °C and milled, were extracted in 800 mL of water under microwave irradiation [a Pyrex flask equipped with a Borosil^{®} Allihn Condensers in a domestic microwave oven modificate]. After extraction [10 min of microwave irradiation at 800 WJ, leaves were filtered, and the solution was dried under pressure. The mixture was filtered with acetone to eliminate the solid residue, and the solution was evaporated under pressure to obtain the crude product. Oleuropein was separated by flash chromatography on [Biotage^{®} Isolera^{™} Systems] equipped with a silica cartridge (Biotage Sfar Silica HC D, CV 15 ml, Capacity 200-2000 g) Flowrate 40 mL/min, UV-VIS (200-800nm) detector and a mixture of CH₂Cl₂/MeOH 8:2 as mobile phase. The purity (≥ 95) was determined by LC/MS/ESI and ¹H-NMR spectroscopy.

LC/MS/ESI (+), m/z 558 (31) [M+NH₄]+, 541 (16) [M+H]+ , 379 (76) [M-Glu+NH₄]+ , 361 (100) [M - Glu + H]+.

¹H-NMR (500 MHz, ACN -d3) d (ppm) = 7.45 (1H, s); 6.69 (1H, d, J = 8.0 Hz); 6.66 (1H, d, J = 1.9 Hz); 6.54 (1H, dd, J1 = 8.0 and J2 = 1.9 Hz); 5.98 (1H, q); 5.76 (1H, s); 4.53 (1H, d, J = 7.9 Hz); 4.18 (1H, m); 4.04 (H1, m); 3.65 (3H, s); 3.88 - 2.00 (sugar protons); 2.71 (2H, m); 2.66 (1H, d, J = 4.0 Hz); 2.37 (1H, d, J = 4.0 Hz); 1.59 (3H, d).

### Mixed lymphocyte reaction (MLR)

MLR were carried out following standard procedures [2]. Briefly, 100,000 MDSCs or TAMs from C57BL/6 mice were co-cultured in 96-well plates with 100,000 lymphocytes from BALB/c mice. Co-cultured cells were treated with 50 µM oleuropein and compared with control vehicle. After 5 days, T-lymphocyte activation, differentiation and proliferation were assessed by flow cytometry. T-cell activation and proliferation markers (Ki-67) and pro-inflammatory cytokine production (IL-2 and IFNγ) were evaluated by flow cytometry in CD4 and CD8 T cells as described before [5].

### Mass spectrometry-based quantitative (shotgun) proteomics and bioinformatics analysis

A unique proteomic experiment was carried out with five biological replicates per sample (m-MDSC, g-MDSC and TAM). Cells were homogenized in a lysis buffer containing 7 M urea, 2 M thiourea 50 mM DTT. The homogenates were spun down at 14000 g for 1 h at 15 °C. Before proteomic analysis, protein extracts were diluted with Laemmli sample buffer in 100 µL, and loaded into a 0.75 mm thick polyacrylamide gel with a 4% stacking gel casted over a 12.5% resolving gel. The run was stopped as soon as the front entered 3 mm into the resolving gel so that the whole proteome became concentrated in the stacking/resolving gel interface. Bands were stained with Coomassie Brilliant Blue, excised from the gel and protein enzymatic cleavage was carried out with trypsin (Promega; 1:20, w/w) at 37 °C for 16 h as previously described [6]. Purification and concentration of peptides was performed using C18 Zip Tip Solid Phase Extraction (Millipore).

### LC-MS/MS

Peptide mixtures were separated by reverse phase chromatography using an UltiMate 3000 UHLPC System (Thermo Scientific) fitted with an Aurora packed emitter column (lonopticks, 25 cm × 75 µm ID, 1.6 µm C18). Samples were first loaded for desalting and concentration into an Acclaim PepMap column (ThermoFisher, 0,5 cm × 300 µm ID, 5 µm C18) packed with the same chemistry as the separating column. Mobile phases were 100% water 0.1% formic acid (FA) (buffer A) and 100% Acetonitrile 0.1% FA (buffer B). Column gradient was developed in a 120 min two step gradient from 5% B to 20% B in 90 min and 20% B to 32% B in 30 min. Column was equilibrated in 95% B for 10 min and 5% B for 20 min. During the whole process, the precolumn was in line with the column and the flow maintained all along the gradient at 300 nl/min. The column temperature was maintained at 40 °C using an integrated column oven (PRSO-V2, Sonation, Biberach, Germany) and interfaced online with the Orbitrap Exploris 480 MS. Spray voltage were set to 2 kV, funnel RF level at 40, and heated capillary temperature at 300 °C. For DDA experiments full MS resolutions were set to 1200,000 at m/z 200 and full MS AGC target was set to Standard with an IT mode Auto. Mass range was set to 375-1500. AGC target value for fragment spectra was set to Standard with a resolution of 15,000 and 3 seconds for cycle time. The intensity threshold was kept at 8E3. Isolation width was set at 1.4 m/z. Normalized collision energy was set at 30%. All data were acquired in centroid mode using positive polarity and peptide match was set to off, and isotope exclusion was on.

### Data Analysis

Raw files were processed with MaxQuant [7] v1.6.17.0 using the integrated Andromeda Search engine [8]. All data were searched against a target/decoy version of the mouse Uniprot Reference Proteome with March 2021 release. First search peptide tolerance was set to 20 ppm, main search peptide tolerance was set to 4.5 ppm. Fragment mass tolerance was set to 20 ppm. Trypsin was specified as enzyme, cleaving after carbamidomethylation of cysteine was specified as fixed modification and peptide N-terminal acetylation, oxidation of methionine, deamidation of asparagine and glutamine and pyro-glutamate formation from glutamine and glutamate were considered variable modifications with a total of two variable modifications per peptide. "Maximum peptide mass" were set to 7500 Da, the "modified peptide minimum score" and "unmodified peptide minimum score" were set to 25 and everything else was set to the default values, including the false discovery rate limit of 1% on both the peptide and protein levels. The Perseus software (version 1.6.14.0) [9] was used for statistical analysis and data visualization. Metascape [10] was used to identify enrichment GO processes, KEGG pathways, reactome gene sets and canonical pathways in our proteomes regulated. Construction of functional interactomes from up- or down-regulated proteins was carried out with the Ingenuity Pathway Analysis Tool (Qiagen) (https://www.qiagen.com/us/products/discovery-and-translational-research/next-generation-sequencing/informatics-and-data/interpretation-content-databases/ingenuity-pathway-analysis/).

### Real-Time Cell Analysis

Cytotoxicity in cell cultures was evaluated by xCELLigence Real-Time Cell Analysis (RTCA) (Roche Diagnostics GmbH, Mannheim, Germany) as described before [3]. Briefly, LLC, H1299, and A549 cells were seeded at a density of 3×10³ cells/well on gold microelectrode-embedded 16-well microplates (E-plates; Roche Diagnostics, Basel, Switzerland) and incubated at 37°C with 5% CO₂. Impedance was recorded at 15 min intervals. Oleuropein (25-250 µM) was added to the culture at seeding time. All incubations were performed in a volume of 100 µl between 0 and 120h. Delta Cell Index values were evaluated by the RTCA-DP software (Roche Diagnostics GmbH). The DCI (Delta Cell index) is used to normalise data. For each well, the Delta Cell Index (DClti) is calculated as the Cell Index CIti at a given time point plus a Delta value. The Delta value is a constant value for each well and is the difference between a reference DCI value and the Cell Index at the Delta time point, as below: DClti = CIti + (DClreference - ClDelta_time). Thus, the Delta Cell Index for all the wells is the reference DCI value at the Delta time point. The default value of the Cl at reference.

### Cell staining and flow cytometry

Surface and intracellular staining were carried out as described before [5] with the following fluorochrome-conjugated antibody clones: AF488 anti-CD49d (R1-2), PE anti-CD4 (GK1.5), APCanti-CD8(53-6.7), brilliant Violet 510^{™} anti-I-A/I-E ( M5/114.15.2,PE/Cyanine7anti-Ly-6G (1A8), and APC anti-CD80 (16-10A1) (Biolegend), FITC anti-IL-12 (C15.6) and BV421 anti-IL-2 (JES6-5H4) (BD Bioscience), APC-Vio ^{®} 770 anti-Ly-6C (REA796), PE REAfinity^{™} anti-F4/80 (REA126), and APC anti-CD68 (REA886) (Miltenyi), PerCP-Cyanine5.5 AntiHuman/Mouse CD11b (M1/70) (Tonbo), and AF488 anti-IFNγ ( Clone XMG1.2) (BD Pharmigen). For human cells, it was used the following fluorochrome-conjugated antibodies: PerCP-Cy5.5-anti-CD11b (integrina alfa M) (M1/70), Violet Fluor 450 anti-CD14 antibody (61D3) (Tonbo), PE anti-CD115 (CSF-1R) (9-4D2-1E4) (Biolegend), PE-Cy^{™}7 anti-CD11c (Clone B-ly6) (BD Bioscience), and APC anti-HLA-DR (REA517) (Miltenyi).

Data were collected using the FACSCanto Flow Cytometer (BD Biosciences) for *ex vivo* and *in vitro* experiments at Navarrabiomed Biomedical Research Center and Cyroflex LX flow cytometer (Beckman Counter). Data were analyzed with Flowjo.

### Myeloid cell samples from NSCLC patients

Twenty-one patients diagnosed with locally advanced or metastatic NSCLC treated with the immune checkpoint inhibitors (ICI) pembrolizumab (anti-PD-1) were recruited for the study. The characteristics of the cohort under study have been described before [11], and this observational study was approved by the Ethics Committee of Clinical Investigations at the University Hospital of Navarre (reference number: PI_2020/115). 11 of the patients were chemoresistant. Blood samples from non-small cell lung cancer (NSCLC) patients were collected prior to treatment and before administration of each immunotherapy cycle.

PBMCs were isolated as described and grown *in vitro* for 24 h in TEXCMACs medium (Miltenyi Biotec) [5]. Myeloid cells were obtained by adherence to plastic for 24 h. Then, 50,000 myeloid cells were seeded per well with 50 µM oleuropein in 96-well plates and incubated for seven days.

### Mouse tumor models and therapies.

C57BL/6 female mice were subcutaneously (s.c) injected with 10⁶ LLC cells or 0.5 × 10⁶ MC38 cells per animal. When tumor growth reached an average diameter of 3.5 mm (day 0), mice received 100 µg of anti-PD-1 mAb (RPMI-14, BioXCell) intraperitoneally (i.p) at days 0, 5, and 13. Control mice received the same volume of saline. For oleuropein, mice received 300 µg oleuropein at days -1, 2, 4, 6, and 8. As negative control, the same volume of saline was injected.

The efficacy of all treatments was evaluated every two days by measuring two perpendicular tumor measurements. The size was calculated using the formula: Size=Length × Width. Mice were humanely sacrificed when tumor size reached -150 mm², or when tumor ulceration or evident discomfort were observed. For rechallenge experiments in the MC38 model, mice that rejected tumors were injected s.c. with 5×10⁵ MC38 cells in the left flank three months after the first tumor inoculation. Naive mice were included as controls and development of tumors was evaluated for two months.

### Statistical analyses

Statistical analyses were performed with the GraphPad Prism 8.3 software package. Cytometry data from *ex vivo* or *in vitro* experiments are normalized against their respective controls (samples not treated with oleuropein). All *in vitro* and ex vivo results from cytometry analysis were normalized against their controls (treated sample value- control sample value). Normality of variables was checked with the Kolmogorov-Smirnov and Shapiro wilk tests (in the case of samples with n<10). Statistical analysis was performed using the paired-tailed Student's t-test (paired dependent t-test), with a significance level of p<0.05. For non-normally distributed data it was used the Wilcoxon matched pairs test.

Tumor measurements of *in vivo* experiments are represented as tumor diameter (mm²) and plotted either as individual data points for one individual mouse or as mean ± SD as indicated in the figure legends. Statistical analysis was performed using one way ANOVA test followed by a Sidak's multiple comparison test at 0.05 level of significance. For in vivo experiments, to compare multiple experimental groups, one-way ANOVA test and Tukey's multiple comparison test were used. Two-tailed Student's t test was applied to compare two experimental groups. Survival of tumor-bearing mice is represented by Kaplan-Meier plots and analyzed by log-rank test. The p values < 0.05 were considered statistically significant.

### RESULTS

### Oleuropein counteracts the immunosuppressive activity of ex vivo differentiated MDSCs and TAMs

The present inventors studied the potential immunomodulatory capacities of the AMPK inhibitor BML-275 and oleuropein over ex vivo-generated TAMCs as single agents and in combination (Fig. 1a).

The present inventors evaluated classical immunosuppressive markers such us STAT3, Vista (vsir), downregulation of antigen presentation markers (MHCII-CD80). In both conditions, these compounds increased MDSCs proliferation and up-regulated classical myeloid activation and antigen presentation markers, such as MHC-II and CD80 (Fig. 1a).

Importantly, oleuropein caused a significant increase of IL-12, a marker for stimulatory myeloid antigen presenting cells.

BML-275 alone or in combination with oleuropein also reduced the expression of CD49d, a specific marker of immunosuppressive MDSCs.

Then, it was also evaluated the effects of BML-275 and oleuropein over TAMs (Fig. 1b) where both compounds induced a significant elevation of cells co-expressing MHC-II and CD80 and upregulated IL-12 production only in the presence of oleuropein. AMPK inhibition in TAMs increased CD68, a maker of M1 phenotype.

To find out whether these treated TAMCs can acquire T cell activation capacities, it was used mixed lymphocyte assays (MLR). Both oleuropein-treated MDSCs and TAMs potently stimulated CD4 T cells as assessed by interferon-gamma (IFNγ) and IL-2 production (Fig. 1c and d). This activation was weaker with the AMPK inhibitor.

Surprisingly, oleuropein polarized MDSCs and TAMs into activated myeloid cells. This reprogramming resulted in enhanced T cell activation capacities.

### Oleuropein causes a molecular reprogramming of functional interactome networks in TAMCs

Oleuropein caused significant changes in TAMC morphology (Fig. 2a). MDSCs acquired a dendritic cell-like morphology (a stimulatory myeloid antigen presenting cells), while TAMs acquired an M1-like phenotype.

To study the mechanisms of action of oleuropein, it was compared the proteome of ex vivo differentiated MDSC subsets and TAMs treated with oleuropein with untreated counterparts as control.

Then, quantitative proteins were used to compare oleuropein-treated with untreated myeloid cells using four or five independent biological replicates. A high number of differentially regulated proteins was identified with a false discovery rate FDR< 1% .Heatmap analysis of all the proteomes ensured that myeloid preparations were homogeneous and grouped by lineage, with oleuropein-treated cells showing distinct proteomic profiles (Fig. 2b).

Ingenuity Pathway Analysis (IPA) was used to identify regulated gene, molecules and pathways. Oleuropein reduced the expression of genes and pathways involved in inflammation such as TREM1 (Fig. 2c). TREM1 is a recently discovered receptor associated with immunosuppressive activity in MDSC and TAMs, which surprisingly was reduced. Cyclic adenosine monophosphate (cAMP) was down-regulated, a pathway also involved in immunosuppression (14).

Interestingly, oleuropein up-regulated LXR/RXR and Fatty acid β-oxidation I signalling in TAMCs subsets (Fig. 2c). Activation of LXR has been shown to reduce MDSC subsets *in vivo* (15) and it has never been shown to be modulated by polyphenols. In addition, IL-9 and RAN signalling were down-regulated, as well as CD40. The main pathways regulating lipid metabolism were altered such as Sirtuin, polyamines and PPAR-α/ RXR-a. Estrogen and insulin signalling were downregulated (Fig. 2c).

In our analyses, therefore, oleuropein changed TAMCs profile by inducing an important biological change at proteomic level. Indeed, oleuropein reprograms the immunosuppressive properties of TAMCs.

Regulated expression of components of the LXR/RXR and fatty acid β-oxidation signalling pathways was observed all TAMCs treated with oleuropein.

Overall, oleuropein caused major global reprogramming of TAMCs by deactivating immunosuppressive pathways.

### Metabolic and functional reprogramming of m-MDSC by oleuropein.

912 proteins differentially regulated were identified in m-MDSC treated with oleuropein (data not shown).

Molecular pathways were constructed with IPA. EIF2, BAG2, sirtuin and granzyme A signalling were activated by oleuropein (Fig. 4a, b). Production of proinflammatory cytokines and FAT10 signalling were down-modulated. (Fig. 4a). In addition, NRF2-mediated oxidative stress response was downregulated. Finally, upstream regulators were identified, highlighting the increase in PTEN and decrease in methylprednisolone (Fig.4b).

In summary, oleuropein demonstrated a potent effect over m-MDSC intracellular regulatory pathways by modulating main regulators involved in MDSCs differentiation.

### Oleuropein interferes with g-MDSC differentiation.

Molecular interactomes were reconstructed in g-MDSC treated with oleuropein, uncovering the upregulation of d mTOR, NET, fmlP and AMPK signaling pathways (Fig. 5a). EIF2, estrogen receptor and granzyme A signalling were downmodulated. Fatty acid beta-oxidation was found activated with a decrease in phospholipids. Upstream regulators were identified, highlighting the increase in Eif6 and decrease in E2f3 up (Fig. 5b). Importantly, Tbk1 and Stat4 proteins that regulates the differentiation and functions of MDSCs were inhibited (16).

Overall, g-MDSC treated with oleuropein drastically changed their cellular programming, acquiring an entirely different proteome. This means that oleuropein prevented the differentiation of myeloid cells to g-MDSC.

### Oleuropein polarizes TAMs towards activated macrophages.

TAMs and MDSCs present intracellular cancer-regulated pathways which are specific for each cell type (9). Hence, it was evaluated the reprogramming of TAMs by oleuropein. Molecular interactions between the significantly regulated proteins were reconstructed by IPA. Oleuropein upregulated EIF2, fmlp, Flt3, and Fcr-mediated phagocytosis signalling (Fig. 5a) but downregulated BAG2, mTOR and Hif1a signalling in TAMs. The upstream regulators were identified, highlighting NUPR1 and GnRH (Fig. 5b).

In summary, oleuropein enhances activated macrophages and the pro-inflammatory activities of macrophages on tumour killing.

### Oleuropein increases phenotypic diversity in myeloid cells from patients with NSCLC.

Phenotypic diversity of myeloid subsets in peripheral blood of cancer patients has been proposed as a biomarker for responses to PD-L1/PD-1 blockade (17). To find out whether oleuropein increased diversity of human myeloid cells, these were isolated from blood samples of NSCLC patients and treated with oleuropein. An increase in CD11b+ cells was observed together with an elevation of the expression of CD115+HLA-DR, CD14+HLA-DR and CD11c+HLA-DR (Fig. 6a), even in the samples from patients showing anticancer resistance. These results indicate a diversification towards monocyte and dendritic cell (DC)-like phenotypes. Similar results were obtained with myeloid cells from healthy donors (Fig. 6b).

### Oleuropein potentiates the antitumor activities of anti-PD-1 mAb in lung cancer model resistant to immunotherapy.

To test antitumor capacities in vivo, LLC cells were implanted subcutaneously into groups of mice and tumors were allowed to grow to an average diameter of approximately 12 mm². This model was chosen because LCC cells are poorly immunogenic and notoriously refractory to PD-L1/PD-1 blockade. Oleuropein was then administered intraperitoneally every other day for eight days. Oleuropein delayed tumor growth, with most of the mice responding to the treatment and significantly increased survival.

To address whether oleuropein could be combined with anti-PD1 immunotherapy to treat LCC-derived tumors, anti-PD1 mAb was systemically administered in combination with intraperitoneal injection of oleuropein. As controls, groups of mice treated with each of the agents singly and saline-treated groups were included. Again, oleuropein significantly delayed tumor growth and increased survival. In contrast, anti-PD-1 mAb on its own did not show any therapeutic effect, confirming that LCC-derived tumors were intrinsically resistant to PD-1 blockade. Importantly, oleuropein and the combination therapy oleuropein /PD-1 blockade demonstrated a significant increase in long-term survival, increased survival up to 20 days and 30 days, respectively.

### Oleuropein improves anti-PD-1 immunotherapy in colorectal cancer.

The efficacy of oleuropein and oleuropein/PD-1 blockade combinations were also tested in a colorectal cancer model. To this end, MC38 cells were subcutaneously implanted in mice and allowed to grow until tumors reached a diameter of about 12 mm². Then, oleuropein was administered intraperitoneally one day prior to anti-PD1 mAb injection. Mice were then treated with four intraperitoneal doses of oleuropein every two days. As in previous experiments, mice treated with anti-PD1 mAb received two additional intraperitoneal doses. The same protocol as the one disclosed in previous section was followed (i.e., 100 µg of anti-PD-1 mAb (RPMI-14, BioXCell) intraperitoneally (i.p) at days 0, 5, and 13. Control mice received the same volume of saline. For oleuropein, mice received 300 µg oleuropein at days -1, 2, 4, 6, and 8. As negative control, the same volume of saline was injected).

Oleuropein significantly delayed the growth of MC38 tumors, leading to a significant increase in survival with a 16% cure rate. This model is susceptible to PD-1 blockade, and as such, anti-PD-1 mAb alone significantly delayed tumor growth with 33% complete regressions and significantly improved long-term survival. Importantly, oleuropein combined with systemic PD-1 blockade demonstrated a very potent antitumor activity, with 66% complete regressions and a significant increase in long-term survival. Mice with complete remissions in the different groups remained tumor-free after MC38 rechallenge, demonstrating immune memory responses.

Summarizing, oleuropein exhibited potent antitumor capacities and most importantly, enhanced anti-PD-1 therapies in models of lung and colon cancer. Importantly, oleuropein improved αPD-1 therapies in a tumor model intrinsically resistant to PD-1 blockade highlights the potentiality of this therapy for cancer treatment. Moreover, these pre-clinical results lead us to put forward oleuropein as a potential adjuvant for clinical trials.

### BIBLIOGRAPHIC REFERENCES

1. Blanco, E. et al., "A Proteomic Atlas of Lineage and Cancer-Polarized Expression Modules in Myeloid Cells Modeling Immunosuppressive Tumor-Infiltrating Subsets", Journal of personalized medicine 2021, 11, doi:10.3390/jpm11060542.
2. Liechtenstein, T. et al., "Anti-melanoma vaccines engineered to simultaneously modulate cytokine priming and silence PD-L1 characterized using ex vivo myeloid-derived suppressor cells as a readout of therapeutic efficacy", Oncoimmunology, 2014, 3, e29178.
3.Gato-Canas et al., "A core of kinase-regulated interactomes defines the neoplastic MDSC lineage", Oncotarget, 2015, 6, 27160-27175, doi:10.18632/oncotarget.4746.
4. Procopio A et al., "Synthesis, biological evaluation, and molecular modeling of oleuropein and its semisynthetic derivatives as cyclooxygenase inhibitors", J Agric Food Chem., 2009;57(23):11161-11167. doi:10.1021/jf9033305
5. Zuazo, M. et al., "Functional systemic CD4 immunity is required for clinical responses to PD-L1/PD-1 blockade therapy", EMBO molecular medicine, 2019, 11, e10293, doi:10.15252/emmm.201910293.
6. Shevchenko, A et al.."In-gel digestion for mass spectrometric characterization of proteins and proteomes", Nat Protoc., 2006, 1, 2856-2860, doi:10.1038/nprot.2006.468.
7.Cox, J. et al., "MaxQuant enables high peptide identification rates, individualized p.p.b.-range mass accuracies and proteome-wide protein quantification", Nature biotechnology, 2008, 26, 1367-1372, doi:10.1038/nbt.1511.
8. Cox, J. et al., "Andromeda: a peptide search engine integrated into the MaxQuant environment", J. Proteome Res., 2011, 10, 1794-1805, doi:10.1021/pr101065j.
9. Tyanova, S. et al., "The Perseus computational platform for comprehensive analysis of (prote)omics data", Nat. Methods 2016, 13, 731-740, doi:10.1038/nmeth.3901.
10. Zhou, Y. et al., "Metascape provides a biologist-oriented resource for the analysis of systems-level datasets", Nature communications, 2019, 10, 1523, doi:10.1038/s41467-019-09234-6.
11. Bocanegra, A. et al., "Potent clinical predictive and systemic adjuvant therapeutic value of plasma fractalkine in PD-L1/PD-1 blockade immunotherapy for lung cancer", MedRxiv 2022.06.16.22276511 2022.
12. Nardi M et al., "Synthesis and antioxidant evaluation of lipophilic oleuropein aglycone derivatives", Food Funct., 2017;8(12):4684-4692
13. M. Oliverio et al., "Semi-synthesis as a tool for broadening the health applications of bioactive olive secoiridoids: a critical review", Natural Product Reports, 2021, 38, 444-469
14. Leone RD, Emens LA, "Targeting adenosine for cancer immunotherapy", J. Immunother. Cancer., 2018, Jun 18;6(1):57. doi: 10.1186/s40425-018-0360-8. PMID: 29914571.
15. Pencheva N. et al., "Broad-spectrum therapeutic suppression of metastatic melanoma through nuclear hormone receptor activation", Cell. 2014 Feb 27;156(5):986-1001. doi: 10.1016/j.cell.2014.01.038. PMID: 24581497.
16. Sun Y. et al., "Targeting TBK1 to overcome resistance to cancer immunotherapy", Nature, 2023,, doi: 10.1038/s41586-023-05704-6. Epub ahead of print. PMID: 36634707.
17. Oliver L. et al., "Mitigating the prevalence and function of myeloid-derived suppressor cells by redirecting myeloid differentiation using a novel immune modulator", J. Immunother. Cancer. 2022 Sep;10(9):e004710. doi: 10.1136/jitc-2022-004710. PMID: 36150744; PMCID: PMC9511656.

## Claims

1. Oleuropein for use in immunotherapy.

2. Oleuropein for use as immunogen in a patient suffering from cancer.

3. Oleuropein for use in inducing immunogenicity to immunosuppressive cells, particularly immunosuppressive tumor associated myeloid cells (TAMCs) population, particularly myeloid-derived suppressor cell (MDSCs) and/or tumor-associated macrophage (TAMs) population.

4. Oleuropein for use according to any of the claims 1-3, wherein oleuropein induces immune cell activation, particularly T cell activation, more particularly CD4 T cell activation.

5. Oleuropein for use according to any of the claims 1-3, wherein the oleuropein increases the sensitivity of the cancer cells for one or more anti-cancer drug(s).

6. Oleuropein for use in a method for the treatment of cancer in combination with one or more anti-cancer drugs, wherein at least one of the anti-cancer drugs is a checkpoint inhibitor.

7. A combination comprising oleuropein and a checkpoint inhibitor anti-cancer drug.

8. A pharmaceutical composition comprising the combination according to claim 7, and one or more pharmaceutically acceptable excipients and/or carriers.

9. A kit of parts comprising:
- Oleuropein,
- A checkpoint inhibitor anti-cancer drug, and
- Optionally carriers or excipients to prepare separate pharmaceutical composition(s).

10. Combination according to claim 7, pharmaceutical composition according to claim 8 or kit of parts according to claim 9, for use in the treatment of cancer.

11. The combination according to claim 7, the pharmaceutical composition according to claim 8, the kit of parts of claim 9 or the oleuropein for use according to claims 5 or 10, wherein the checkpoint inhibitor is selected from anti-PD-1 or PD-L1 inhibitors.

12. The oleuropein for use according to any of the claims 2, 4-6 and 10-11, wherein the cancer is selected from the group consisting of: liver cancer, stomach cancer, colon cancer, breast cancer, lung cancer, non-small cell lung cancer, bone cancer, pancreatic cancer, skin cancer, head or neck cancer, cutaneous or intraocular melanoma, uterine cancer, ovarian cancer, colorectal cancer, small intestine cancer, rectal cancer, anal cancer, fallopian tube cancer, endometrial cancer, cervical cancer, vaginal cancer, vulva cancer, Hodgkin's disease, esophageal cancer, small intestine cancer, lymph node cancer, bladder cancer, gallbladder cancer, endocrine cancer, thyroid cancer, parathyroid cancer, adrenal cancer, soft tissue sarcoma, urethra cancer, penis cancer, prostate cancer, adenocarcinoma, chronic or acute leukemia, lymphocytic lymphoma, bladder cancer, kidney or ureter cancer, renal cell carcinoma, renal pelvic carcinoma, central nervous system tumor, primary CNS tumor, spinal cord tumor, brainstem glioma, and pituitary adenoma.; particularly, lung cancer.

13. The oleuropein for use according to any of the claims 2, 5-6, 10-12, wherein cancer is at least partially therapy-resistant.

14. The oleuropein for use according to any of the preceding claims 5-6, 10-12, which comprises the simultaneous, separate or consecutive administration of the oleuropein and the one or more anti-cancer drug(s).

15. The oleuropein for use according to any of the preceding claims 5-6, 10-12, wherein the oleuropein and the one or more anti-cancer agents are administered separately, together with one or more pharmaceutically acceptable carriers or excipients.
